# EUROPEAN PATENT APPLICATION

(11) **EP 2 047 814 A1**
(43) Date of publication of application: **15.04.2009**
(21) Application number: 08164848.7
(22) Date of filing: 23.09.2008
(51) Int. Cl.: A61B 17/70

(54) **Rod assembly for dynamic posterior stabilization**

(30) Priority: 16.06.2008 US 139871; 12.10.2007 US 979489 P
(71) Applicant: Aesculap AG, 78532 Tuttlingen (DE)
(72) Inventor: Szczesny, Spencer, Bethlehem, PA 18018 (US)
(74) Representative: Hoeger, Stellrecht & Partner Patentanwälte

(57) **Abstract**

An assembly (20) for dynamic stabilization of the spine includes a rod (30) having a first end (34) having a first diameter and a second end (36) having a second diameter smaller than the first diameter. A hollow housing (40) forms a passage that receives the second end (36) of the rod. An elastic member (50) connects the first end of the rod (30) with the housing (40). The assembly (20) includes or is operable with a first pedicle screw (P) and a second pedicle screw (P), each pedicle screw (P) having a rod receiving slot. The first end (34) of the rod is supported in the rod receiving slot of the first pedicle screw, and the housing (40) is supported in the rod receiving slot of the second pedicle screw.

## Description

### RELATED APPLICATIONS

This non-provisional application claims the benefit of priority to U.S. Provisional Application No. 60/979,489, filed October 12, 2007 and U.S. Patent Application No. 12/139,871 filed June 16, 2008, the entire contents of which are incorporated by reference herein for all purposes.

### FIELD OF THE INVENTION

The present invention relates generally to treatment of the spine, and more particularly to a method and apparatus for dynamic stabilization of the lumbar spine.

### BACKGROUND

Historically, spinal fusion was the surgical therapy of choice for patients with spine disorders. Spinal fusion involves the replacement of the intervertebral disc with a bone graft and often posterior fixation using bone screws and solid titanium rods. The stabilization provided by the screws allows the bone graft to grow between the two vertebrae. This fuses the vertebrae together as one solid piece of bone.

Spinal fusion is capable of providing local stability at the motion segment to relieve pain. Nevertheless, spinal fusion can create or exacerbate other problems, including damage to nearby discs. Because a fused joint cannot bend, the spine loses mobility at that joint. Nearby disc joints must make up for the lost mobility by moving through a larger range of motion. The larger range of motion is often larger than the natural range of motion for the nearby discs. This can cause the nearby disc joints to become damaged or degenerate at an accelerated rate, leading to complications and the need for more surgeries. The process of degeneration and disease of intervertebral discs neighboring a fused disc is sometimes referred to as Adjacent Segment Degeneration (ASD).

An alternative to spinal fusion is dynamic stabilization of the spine, in conjunction with or without fusion. In dynamic stabilization, the stiffness of a diseased or damaged spine is increased in various directions of movement. Some controlled movement is still allowed, especially in flexion, extension and lateral bending. This form of therapy provides enough stability to reduce pain, yet also allows some motion to delay or prevent further degeneration of neighboring discs. Historically, dynamic stabilization has been performed by implanting a solid rod made of a flexible material, such as polyurethane or polyetheretherketone (PEEK). These rods are largely perceived to allow bending of the spine. A biomechanical analysis of the performance of these rods demonstrates that this perception is not true, and that solid flexible rods have the unexpected result of constraining motion.

### SUMMARY OF THE INVENTION

Adverse effects of spinal surgery, such as ASD and other complications, can be alleviated to a large extent by assemblies for dynamic stabilization of the spine, in accordance with the present invention.

In a first aspect of the invention, an assembly for dynamic stabilization of the spine includes a rod having a first end with a first diameter and a second end with a second diameter smaller than the first diameter. A hollow housing forms a passage that receives the second end of the rod. An elastic member connects the first end of the rod with the housing.

In a second aspect of the invention, an assembly for dynamic stabilization of the spine includes a rod having a first end with a first diameter and a second end with a second diameter smaller than the first diameter. A hollow housing forms a passage that receives the second end of the rod. An elastic member connects the first end of the rod with the housing. The assembly further includes a first pedicle screw and a second pedicle screw, each pedicle screw having a rod receiving slot. The first end of the rod is supported in the rod receiving slot of the first pedicle screw, and the housing is supported in the rod receiving slot of the second pedicle screw.

In a third aspect of the invention, an assembly for dynamic stabilization of the spine includes a rod having a first end with a first diameter and a second end with a second diameter smaller than the first diameter. A sleeve is coupled to the rod and circumscribes at least a portion of the second end of the rod. The sleeve includes a cylindrical housing and an elastic section connected between the cylindrical housing and the first end of the rod. The elastic section is expandable in a direction parallel to the longitudinal axis of the rod.

### BRIEF DESCRIPTION OF THE DRAWING FIGURES

The foregoing summary and the following description will be better understood when reviewed with the drawing figures, of which:
FIG. 1 is a schematic view of a conventional solid flexible rod used in dynamic stabilization under a first condition;
FIG. 2 is a schematic view of a conventional solid flexible rod used in dynamic stabilization under a second condition;
FIG. 3 is an elevation view of a rod assembly in accordance with an exemplary embodiment of the present invention;
FIG. 4 is a perspective view of a pair of rod assemblies in accordance with exemplary embodiments of the present invention, shown schematically with a portion of a human spine;
FIG. 5 is a side view of a rod assembly in accordance with an exemplary embodiment of the present invention, shown schematically with a portion of the spine;
FIG. 6 is a side view of an alternate rod assembly in accordance with an exemplary embodiment of the present invention, shown schematically with a portion of the spine;
FIG. 7 is an elevation view of the rod assembly of FIG. 3, with a portion broken away to illustrate internal features of the assembly;
FIG. 8 is an enlarged view of a portion of the rod assembly of FIG. 7;
FIG. 9 is an exploded elevation view of the rod assembly of FIG. 3;
FIG. 10 is a cross-sectional view of a component illustrated in FIG. 9;
FIG. 11 is an elevation view of the rod assembly of FIG. 3 with additional components in accordance with the invention;
FIG. 12 is an exploded elevation view of the rod assembly of FIG. 11;
FIG. 13 is an elevation view of a rod component in the rod assembly of FIG. 3;
FIG. 14 is an elevation view of the rod component of FIG. 13, rotated 90 degrees;
FIG. 15 is a top view of a collar component in the rod assembly of FIG. 3;
FIG. 16 is a perspective view of the collar component of FIG. 15;
FIG. 17 is a perspective view of an elastic member in accordance with the one exemplary embodiment of the present invention;
FIG. 18 is an elevation view of a housing component of the rod assembly of FIG. 3;
FIG. 19 is an elevation view of the housing component of FIG. 18, rotated 90 degrees;
FIG. 20 is a cross-sectional view of the housing component of FIG. 18, taken through line 20-20;
FIG. 21 is an elevation view of an alternate rod component in accordance with an exemplary embodiment of the invention;
FIG. 22 is an elevation view of another alternate rod component in accordance with an exemplary embodiment of the invention;
FIG. 23 is an elevation view of components of another exemplary rod assembly in accordance with the invention, with a portion shown in cross section;
FIG. 24 is a perspective view of another exemplary rod assembly in accordance with the invention, shown schematically with a portion of a human spine; and
FIG. 25 is an exploded view of the rod assembly of FIG. 24.

### DETAILED DESCRIPTION OF PREFERRED EMBODIMENTS OF THE INVENTION

Dynamic stabilization in accordance with the present invention deviates from pre-conceived designs that utilize solid flexible rods anchored at both ends. Embodiments of the present invention utilize a rod assembly having different sections along its length, with each section having different components and cross-sections. The different sections vary the kinetic properties along the length of the rod assembly. The rod assembly may be implanted over a single disc, or over a series of adjacent disc locations. By way of example, a rod assembly in accordance with the invention may include a relatively stiff solid section to be connected over a damaged disc, and more flexible sections to be connected over discs that are adjacent or in proximity to the damaged disc (i.e., "neighboring discs"). The flexible sections provide a more natural range of motion at neighboring discs, while still limiting the motion to a safe range to prevent onset of ASD. The flexibility and range of motion of the flexible sections can be gradually increased as the rod extends away from the disc of concern.

In accordance with an exemplary embodiment of the present invention, a rod assembly includes interconnected components that allow controlled movement of vertebrae in multiple directions. Rather than using a single homogeneous flexible component, such as a solid flexible rod, to provide flexibility in all directions, the preferred embodiment of the present invention uses a number of different dynamic components. Each dynamic component is responsible for contributing to flexibility in one or more directions, while restricting motion in other directions. In this configuration, the assembly has dynamic flexibility both in terms of bending and in terms of axial expansion (stretching). Unlike solid one-piece rods, axial expansion is not dependent on the same component(s) that provides bending motion. Accordingly, bending flexibility can be adjusted without influencing the assembly's ability to expand and contract in the axial direction. A number of embodiments are anticipated, and include the following exemplary embodiments without limitation.

In a first embodiment, an assembly for dynamic stabilization of the spine includes:
a rod comprising a first end having a first diameter and a second end having a second diameter smaller than the first diameter;
a hollow housing forming a passage that receives the second end of the rod; and
an elastic member connecting the first end of the rod with the housing.

In a second embodiment, an assembly for dynamic stabilization of the spine includes:
a rod comprising a first end having a first diameter and a second end having a second diameter smaller than the first diameter;
a hollow housing forming a passage that receives the second end of the rod;
an elastic member connecting the first end of the rod with the housing;
a first pedicle screw having a rod receiving slot, the first end of the rod being supported in the rod receiving slot of the first pedicle screw; and
a second pedicle screw having a rod receiving slot, the housing being supported in the rod receiving slot of the second pedicle screw.

In a third embodiment, an assembly for dynamic stabilization of the spine includes:
a rod having a longitudinal axis, the rod comprising:
   a first end having a first diameter; and
   a second end having a second diameter smaller than the first diameter; and
a sleeve coupled to the rod that circumscribes at least a portion of the second end of the rod, the sleeve comprising:
   a cylindrical housing; and
   an elastic section connected between the cylindrical housing and the first end of the rod, the elastic section being expandable parallel to the longitudinal axis of the rod.

The use of separate independent components provides a controlled flexibility that is not available with assemblies that feature a single homogeneous element, such as a solid flexible rod. When a solid rod is connected to two adjacent vertebrae by pedicle screws, bending motion between the vertebrae often requires axial expansion and contraction of the rod. Bending of the spine often requires a certain amount of axial elongation of the rod, in addition to bending. A solid flexible rod is typically too stiff to allow for expansion or contraction, even though it has high bending flexibility. The solid body construction of the rod usually has high tensile strength and is not designed to stretch in an axial direction. The need for axial expansion and contraction is therefore ignored by solid rod designs. This can be visualized by a biomechanical analysis of a conventional solid or homogeneous rod implanted on a spine.

Preferably, the elastic member circumscribes at least a portion of the rod between the first end and the second end.

Advantageously, the elastic member comprises a coiled spring.

Preferably, at least one of the rod and the housing comprises a spiral-shaped groove adapted to receive an end portion of the coiled spring.

Advantageously, the assembly comprises at least one locking collar connecting the elastic member with one of the rod and the housing.

Preferably, the elastic member comprises a first end and a second end opposite the first end, the assembly further comprising a first locking collar coupling the first end of the elastic member with the rod and a second locking collar coupling the second end of the elastic member with the housing.

Advantageously, the passage comprises an inner wall that is offset from the axis of the rod by an angle between about 0 degrees and about 15 degrees.

Preferably, the angle is about 7 degrees.

Advantageously, the angle is about 10 degrees.

Preferably, the assembly comprises a flexible sheath extending over the elastic member between the first end of the rod and the housing.

Advantageously, the assembly further comprises:
a first pedicle screw having a rod receiving slot, the first end of the rod being supported in the rod receiving slot of the first pedicle screw; and
a second pedicle screw having a rod receiving slot, the housing being supported in the rod receiving slot of the second pedicle screw.

Preferably, the at least one locking collar comprises a bore that receives a portion of the elastic member.

Advantageously, the elastic member comprises a coiled spring and the bore of the locking collar comprises a groove receiving a portion of the coiled spring.

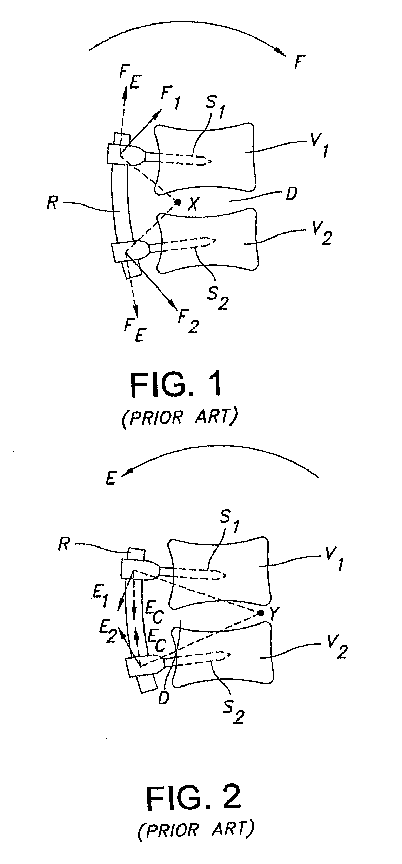

Referring to FIGS. 1 and 2, a flexible solid R is schematically shown as it would be implanted over a disc space D to dynamically stabilize the disc. Rod R is implanted on the posterior of the spine by a pair of pedicle screw assemblies S₁ and S₂. Screw assembly S₁ is anchored in a superior vertebral body V₁, and screw assembly S₂ is mounted in an inferior vertebral body V₂.

FIG. 1 illustrates the biomechanics of rod R during flexion, the direction of which is indicated by curved arrow F. During flexion, the motion of vertebral bodies V₁ and V₂ impart forces to pedicle screws S₁ and S₂ and urge the pedicle screw heads to pivot about a point. The approximate location of this point is indicated at point X. The head of screw S₁ is urged in the direction noted by arrow F₁, and the head of screw S₂ is urged in the direction noted by arrow F₂. The forces on screws S₁ and S₂ each include a component of expansion force F_{E}, which is transferred to rod R. Because solid rod R is not designed to be elongated, the expansion forces are resisted, and flexion is very limited or prevented. Accordingly, the expected mobility of the vertebral bodies by virtue of using a bendable rod is not attained during flexion, leaving neighboring discs prone to ASD or other complications.

FIG. 2 similarly illustrates the biomechanics of rod R during extension, the direction of which is indicated by curved arrow E. During extension, the motion of vertebral bodies V₁ and \/₂ impart forces to pedicle screws S₁ and S₂ and urge the pedicle screw heads to pivot about a point. The approximate location of this point is indicated at point Y. The head of screw S₁ is urged in the direction noted by arrow E₁, and the head of screw S₂ is urged in the direction noted by arrow E₂. The forces on screws S₁ and S₂ each include a component of compression force E_{C}, which is transferred to rod R. Because solid rod R is not designed to be axially compressed, the compression forces are resisted, and extension is very limited or prevented. As with flexion, the expected mobility of the vertebral bodies by virtue of using the bendable rod is not attained during extension, leaving neighboring discs prone to ASD or other complications.

Based on the foregoing, the use of solid flexible rods in dynamic stabilization can have the unexpected result of providing fixation at locations where mobility is expected. The use of solid flexible rods, or any rods that do not permit elongation and contraction in addition to bending, can fall short of matching the true biomechanics of vertebral motion.

Embodiments of the invention address the biomechanics of vertebral motion by allowing not only bending of the rod assembly, but also by allowing axial expansion and contraction of the rod assembly. In particular, the preferred embodiments allow a controlled amount of linear expansion and contraction of the rod assembly between the pedicle screw heads. The preferred embodiments also allow controlled angular changes between the two pedicle screws to permit a defined amount of angular motion between vertebrae.

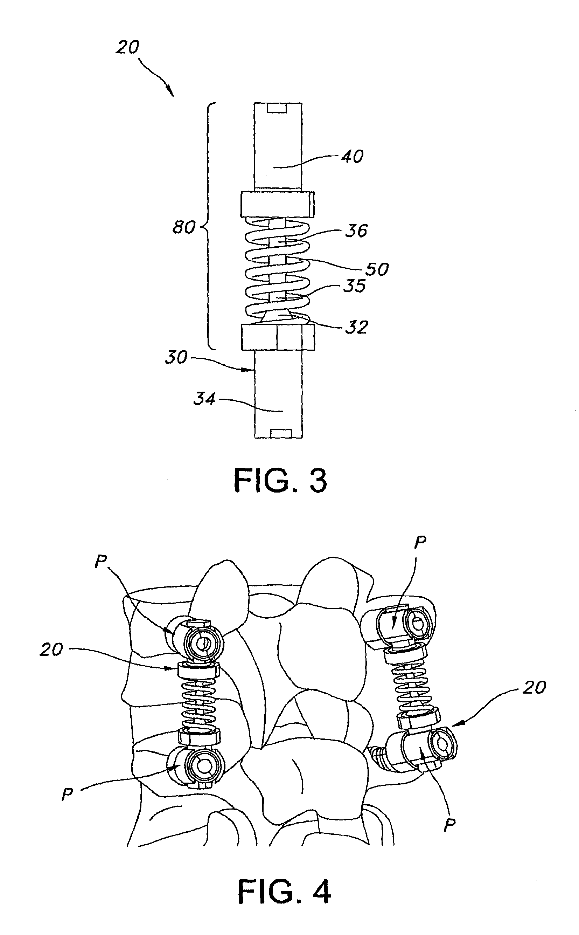

Referring now to FIG. 3, an exemplary embodiment of a rod assembly 20 is shown in accordance with the present invention. Rod assembly 20 includes a variable-diameter rod 30, a housing 40 and an elastic member 50 that interconnects the rod and the housing. The variable-diameter rod 30 has an elongated body 32 having a relatively large diameter end 34 and a relatively small diameter end 36. The large diameter end 34 and small diameter end 36 are separated by a transition portion 35 located at a midsection of the rod 30. Housing 40 and elastic member 50 are coupled together and surround the small diameter end 36, collectively forming a sleeve 80. Sleeve 80 includes an axially expandable section in elastic member 50, and a non-expandable section in housing 40.

Housing 40 has a hollow passage that receives the small diameter end 36 of variable-diameter rod 30. Rod 30 and elastic member 50 are both flexible, allowing assembly 20 to bend in numerous directions. The flexibility of elastic member 50 also allows assembly 20 to linearly expand or lengthen under tension, and linearly contract or shorten under compression.

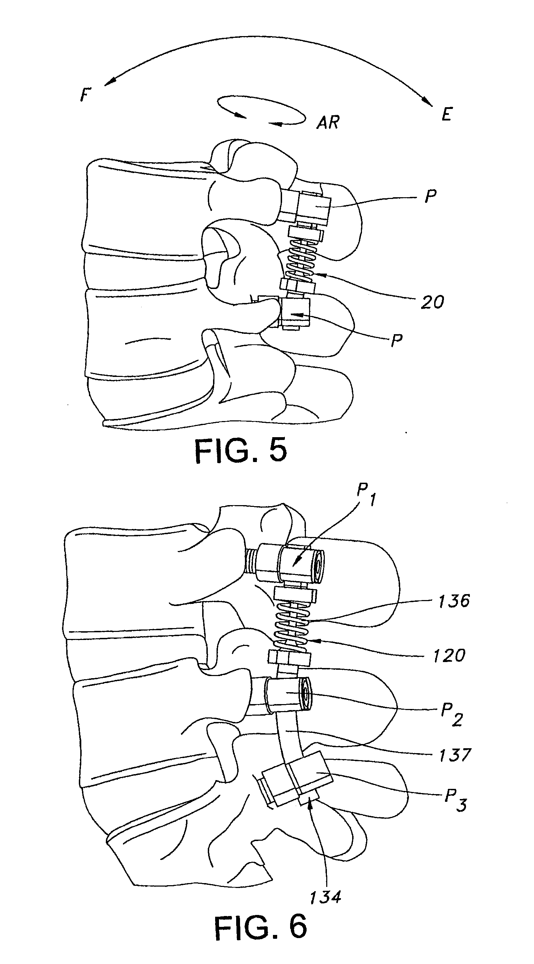

Each end of assembly 20 is configured to be secured in the rod receiving body of a bone anchor. It is contemplated that the assembly 20 may be used with various bone anchors. FIGS. 4 and 5 provide a schematic view of two assemblies 20 attached to pedicle screws "P" in the lumbar spine. Large diameter end 34 of rod 30 is secured in a rod-receiving slot of a first pedicle screw and housing 40 is secured in a rod-receiving slot of a second pedicle screw. In this arrangement, large diameter section 34 and housing 40 form two support sections or "gripping sections" on assembly 20 that can be secured to pedicle screws.

FIG. 6 shows a schematic view of an alternate rod assembly 120 that is used in a "topping-off" construct. In topping-off, a rigid rod section is placed over a disc of concern, such as a partially damaged disc that requires rigid stabilization or fusion. The rigid rod section is then topped-off, or adjoined to a more flexible rod section that extends over an adjacent disc. In this construct, motion at the disc of concern is substantially minimized, and motion at the adjacent disc is limited to a range of motion that prevents ASD. Depending on the desired stabilization, this limited range of motion at the adjacent disc may mimic the normal range of motion at that disc.

Rod assembly 120 is connected over three vertebrae by a superior pedicle screw P1, a middle pedicle screw P2 and an inferior pedicle screw P3. In this application, large diameter end 134 of the rod 130 has an elongated section 137. Because of its larger diameter, section 137 is stiffer in comparison to the smaller diameter section 136 of rod 130. The stiffer, more rigid section 137 extends over a disc "A" to be fused, and the more flexible section 136 extends over a disc "B" to be topped-off. When disc A is fused, adjacent discs like disc B will be subject to greater displacement to make up for the loss of mobility in disc A. Dynamic rod assembly 120 limits or prevents the onset of ASD in disc B by restricting the range of motion of disc B. Although the range of motion at disc B is restricted, there is still sufficient flexibility for elongation, contraction and bending of the rod assembly at that section, allowing some range of motion that mimics the natural behavior of the spine at that location. The dynamic behavior of the rod section between superior screw P1 and middle screw P2 does not disrupt the relatively fixed condition of the assembly between middle screw and inferior screw P3. Axial expansion, contraction and bending motion of the rod assembly between P1 and P2 does not transfer to the rod section between the middle screw and inferior screw P3 because of the stiffer solid rod section 137. In this arrangement, controlled deflection of rod 130 is isolated over disc B between superior screw P1 and middle screw P2.

Dynamic rod assemblies in accordance with the invention provide flexibility along certain selected axes while limiting or preventing displacement along other axes. The various directions of movement are illustrated in FIG. 5. FIG. 5 is a schematic view of a side profile of the spine. Double-ended arrow "F-E" illustrates flexion (direction "F") and extension (direction "E"). Medial-lateral bending is normal to the figure, or in the plane perpendicular to the plane passing through double-ended arrow F-E. Axial rotation is illustrated by double-ended arrow "AR".

The components of rod assembly 20 each provide a moderate degree of flexibility for motion in certain directions. Rod 30 provides resistance to translation in directions perpendicular to the axis of the rod, or "horizontal translation", while the elastic member 50 allows for flexion, extension and medial-lateral bending. Elastic member 50 also allows a limited amount of axial elongation, or "vertical translation" along the axis of rod 30, and a small amount of rotation about the axis of the rod. The thickness, stiffness and type of material selected for elastic member 50 can be adjusted to provide a desired range of vertical translation and axial rotation.

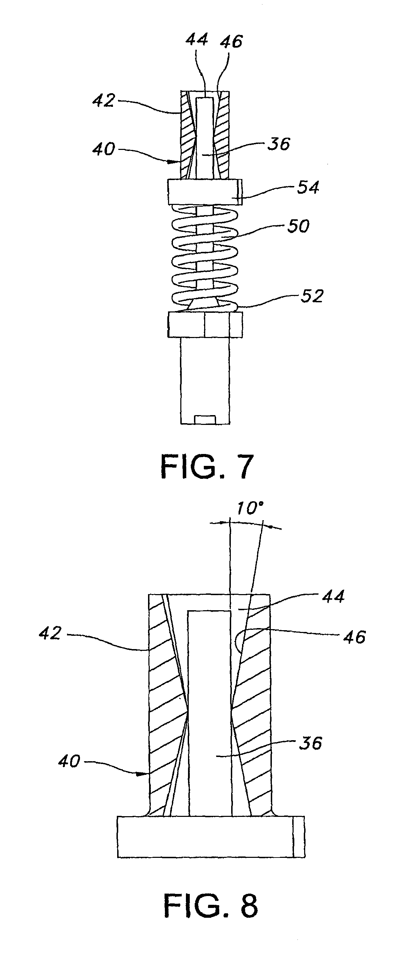

Housing 40 permits a limited range of displacement of small diameter end 36 of rod 30 relative to the housing. Referring to FIGS. 7 and 8, housing 40 includes a hollow body 42 that forms a pivot chamber 44. Pivot chamber 44 is surrounded by an hour glass-shaped inner wall 46. Rod 30 can slide or pass through the chamber 44 in an axial direction but can not move or translate horizontally in a direction normal to the rod axis. As such, the superior and inferior vertebrae are prevented from moving solely through horizontal translation with respect to one another, i.e. linear motion perpendicular to the rod axis. Rod 30 is permitted to bend in the anterior direction, posterior direction or medial-lateral direction, however. That is, chamber 44 permits a limited range of pivoting of the small diameter end 36 relative to housing 40 to facilitate bending of the small diameter end. The maximum pivot angle is limited by the angle of the hour glass-shaped inner wall relative to the axis of the rod. This angle is preferably between about 0 degrees and about 15 degrees. Larger angles outside of this range, which offer a larger range of motion, may also be appropriate. In FIG. 8, the maximum angle is shown as 10 degrees in any direction. The inner wall 46 acts as a pivot stop for the small diameter end 36.

Thus far, elastic member 50 has been shown in the form of a coil spring. A coil spring configuration is advantageous in that it provides flexibility in numerous directions and facilitates a secure connection with locking collars, to be described in more detail below. Nevertheless, a number of configurations for the elastic member may provide the desired degree of flexibility while facilitating secure connections. For example, a bellows-type component may be used to flexibly connect the large diameter end 34 with housing 40. Therefore, the coil spring shape is not the only configuration that is contemplated for use with the present invention.

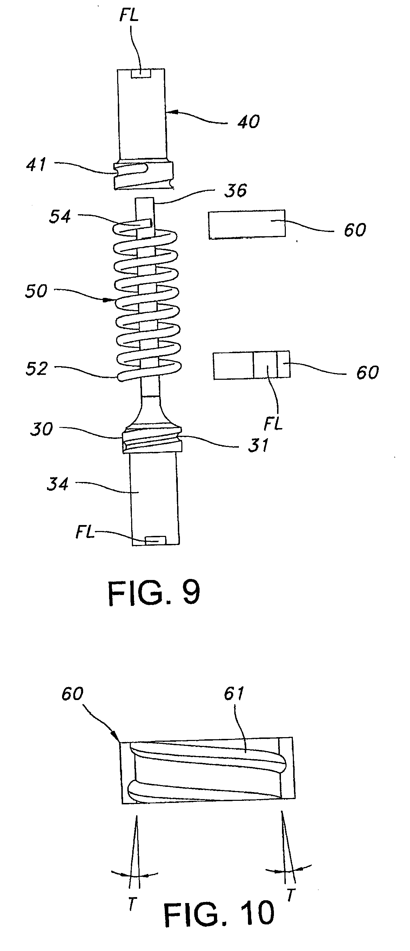

The rod 30, housing 40 and elastic member 50 may be interconnected in a number of ways. For example, a first end 52 of elastic member 50 may be joined to large diameter end 34 of rod 30 by an adhesive or welding, and a second end 54 of the elastic member may similarly be joined to housing 40 by an adhesive or welding. Referring now to FIGS. 9 and 10, elastic member 50 is attachable to rod 30 and housing 40 by locking collars 60. Each locking collar 60 forms a hollow ring with an interior connector surface that couples an end of elastic member 50 to either rod 30 or housing 40. The interior of each locking collar 60 has a spiral groove 61 that matches the pitch of coil spring 50. A similar spiral groove 31 is cut into large diameter end 34 of rod 30, and another spiral groove 41 is cut into an end of housing 40.

To assemble the components, a first locking collar 60 is threaded over end 52 of elastic member 50 and advanced to a middle section of the elastic member. The second locking collar 60 is threaded over the opposite end 54 of elastic member 50 and advanced to a middle section of the elastic member. End 52 of elastic member 50 is then threaded into groove 31 of rod 30, and end 54 of elastic member is threaded into groove 41 of housing 40. Once elastic member 50 is secured to the rod 30 and housing 40, the first locking collar 60 is rotated and moved out from the middle section of the elastic member until the locking collar surrounds end 52 of the elastic member where it joins rod 30. The second locking collar 60 is similarly rotated and moved along the elastic member 50 in the opposite direction until the locking collar surrounds end 54 of the elastic member where it joins housing 40.

The spiral grooves 31, 41 and 61 in rod 30, housing 40 and locking collars 60, respectively are each cut with a slight taper. That is, the diameters of the grooves gradually increase along the length of the respective component. Preferably, this taper is about 3 to about 4 degrees relative to the longitudinal axis of the component. Other taper angles may also be satisfactory. A taper angle "T" is shown for the collar groove 61 in FIG. 10. The minimum diameter of groove 61 (i.e. the diameter of the groove at the top of collar 60 shown in FIG. 10) is sufficiently larger than the diameter of elastic member 50 to allow the collar to thread and move easily over the elastic member. This arrangement allows the collars to fit loosely over the elastic member, and prevent the collar from possibly deforming the shape of the elastic member as it is threaded over the elastic member.

As the ends 52, 54 of elastic member 50 are threaded onto rod 30 and housing 40, the gradually tapering grooves 31 and 41 force the ends of the elastic member to expand radially outwardly. Collars 61 are threaded onto elastic member 50 so that the diameters of the grooves 61 increase outwardly toward the ends of the elastic member, in conformance with the expanded diameter of the elastic member at its ends. As the collars 61 pass over the expanded ends of elastic member 50, the walls of groove 61 engage the expanded ends of the elastic member and form a positive interference lock with the elastic member 50. The expanded ends of elastic member 50 cause the collar 60 to grab and clamp down on the ends of the elastic member and firmly secure it to the rod 30 and housing 40.

Flats "FL" are provided on the exterior diameters of rod 30, housing 40 and locking collars 60 so that the collars can be securely twisted onto elastic member 50 with a tool. To prevent locking collars 60 from loosening, the locking collars can be welded or brazed to the surface of both the housing 40 and rod 30. For example, one or more spot welds may be added at the junction of the collars and elastic member to prevent the collars from loosening. Alternatively, locking collars 60 can be pinned to housing 40 and rod 30.

The material chosen for housing 40 and rod 30 is ideally a cobalt-chrome alloy. This is a strong material that makes a good metal-on-metal bearing surface. Alternatively, rod 30 may be made from a biocompatible plastic if more flexibility in bending is desired. Elastic member 50 can be made out of a metal like titanium or cobalt-chrome, or a biocompatible plastic. The decision depends on the desired stiffness, fatigue life of the spring, and other considerations. Locking collars 60 can also be made of titanium, cobalt-chrome, or a biocompatible plastic, although if it is to be welded, then it is preferably a metal.

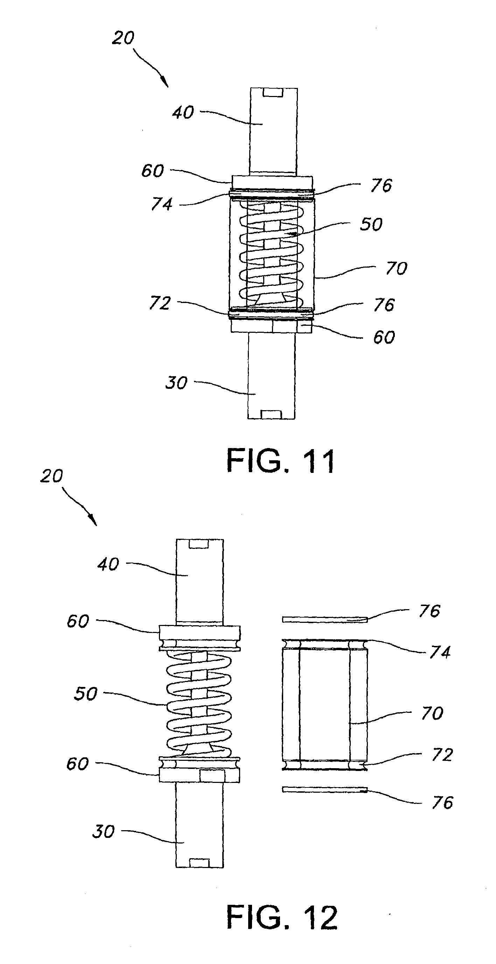

Referring now to FIGS. 11 and 12, dynamic rod assembly 20 optionally includes a flexible sheath 70. Sheath 70, which may be formed of a flexible polyurethane, can be added to assembly 20 to ensure that there is no tissue ingrowth into the area of the elastic member 50. This is both to maintain the dynamism of the elastic member 50 and protect local tissue from irritation. Flexible sheath 70 has a first end 72 oriented toward large diameter end 34 of rod 30, and a second end 74 oriented toward housing 40. First and second ends 72 and 74 of sheath 70 are coupled with locking collars 60 by wire rings 76. Alternatively, sheath 70 may be connected with collars 60 by ultrasonic welding, adhesives or other suitable attachment means.

In some patients, tough, fibrous scar tissue may form around the implant and bond to the exterior of sheath 70. This scar tissue is often much stiffer than flexible sheath 70, and can prevent the sheath from stretching. If this happens, the scar tissue can limit or prevent the rod assembly 20 from bending or elongating. To avoid this issue, it may be desirable to use a sheath formed of a bendable but structurally stable plastic material that is attached to the assembly at only one of its ends. This allows the sheath to slide over the exterior of the assembly as the elastic member 50 stretches and compresses, rather than stretch or collapse. Scar tissue that bonds to the sheath will slide with the sheath.

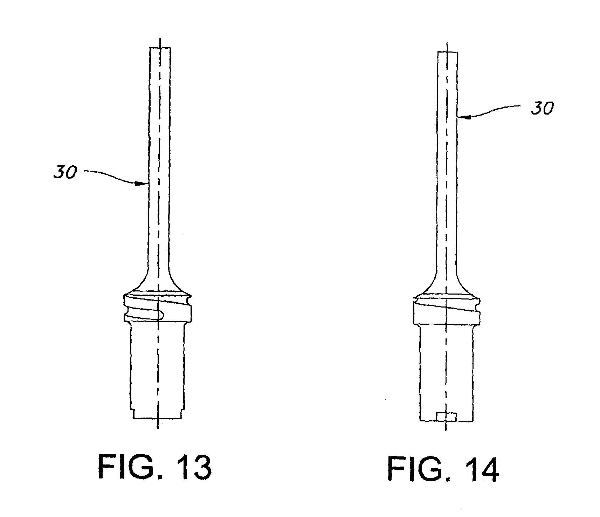

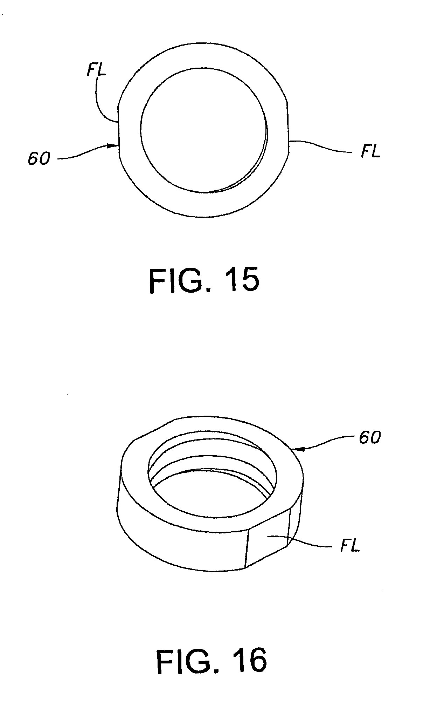

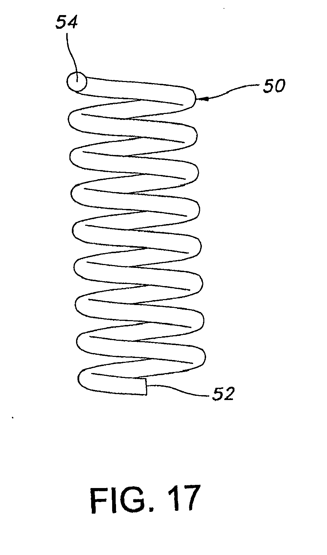

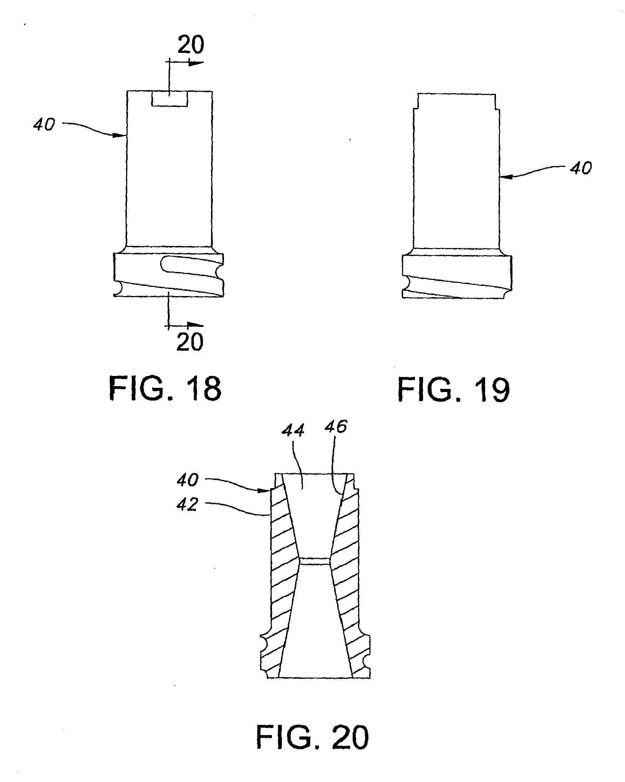

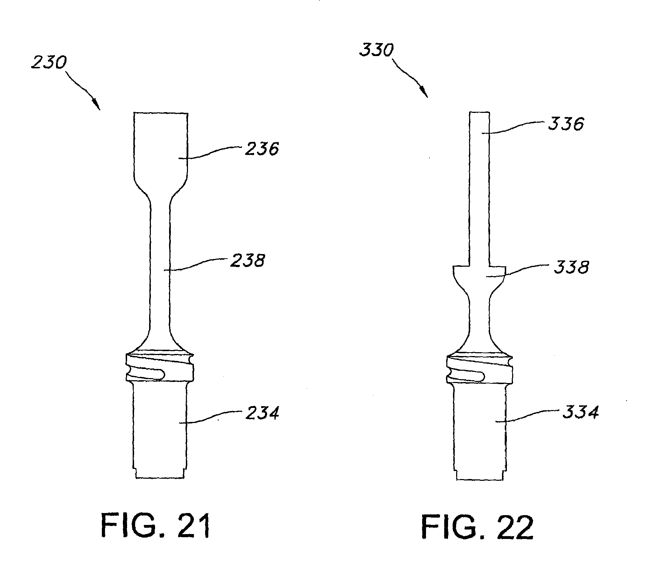

Referring to FIGS. 13-22, components of the rod assembly 20 are shown in additional detail. FIGS. 13 and 14 provide perspective views of variable diameter rod 30. It will be noted that variable diameter rod 30 may assume a number of other configurations, and need not be limited to the configuration shown. Referring to FIG. 21, for example, a rod 230 includes a first end 234 having a first diameter, a second end 236 having a second diameter, and a midsection 238 having a third diameter that is smaller than the first and second diameters, similar to an hour glass. The midsection would provide most of the rod's flexibility in such a configuration. FIGS. 15 and 16 provide a top view and perspective view, respectively, of locking collar 60. FIG. 17 is an elevation view of elastic member 50. FIGS. 18 and 19 are front and side elevation views of housing 40, respectively. FIG. 20 is a cross-sectional view of housing 40 taken through line 20-20 of FIG. 18.

It may be preferable to limit the amount of axial compression and axial expansion of the elastic member, so as to ensure a longer fatigue lifetime of the elastic member. This may be accomplished by introducing one or more stops in the assembly. Referring to FIG. 22, another exemplary rod 330 includes a first end 334 having a first diameter, a second end 336 having a second diameter, and a midsection 338 having a third diameter that is larger than the second diameter yet smaller than the first diameter. The midsection 338 acts as a stop to limit axial compression of an elastic element, such as an elastic spring, to a pre-defined range by blocking the sliding motion of the housing over the second end 336 of the rod. The stop protects the spring from excessive loads that could damage the spring or cause excessive wear.

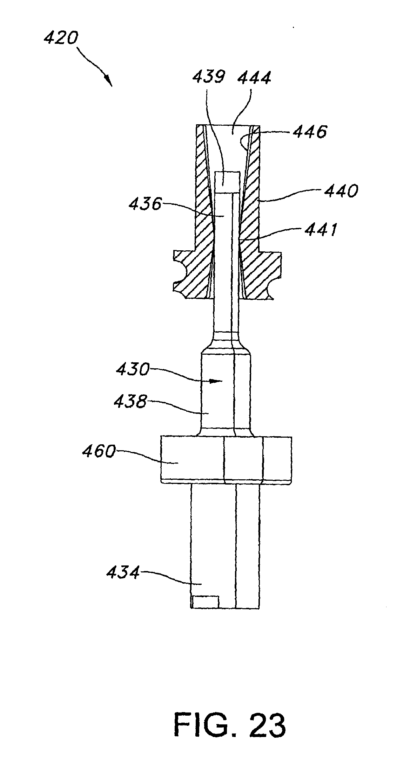

Referring now to FIG. 23, another exemplary rod assembly 420 is shown in accordance with the invention. Rod assembly 420 is similar in many respects to the assembly 20 discussed previously, and includes an elastic member and a pair of collars 460 for securing the elastic member to a rod 430 and housing 440. The elastic member and one of the collars 460 are not shown, so as to allow illustration of features in the interior of housing 440. It will be understood that the omitted elastic member and collar may be arranged in the same manner shown on assembly 20. Rod 430 includes a first end 434 having a first diameter, a second end 436 having a second diameter, and a midsection 438 having a third diameter that is larger than the second diameter yet smaller than the first diameter. Midsection 438 acts as a stop to limit axial compression of elastic element to a pre-defined range by blocking the sliding motion of housing 440 toward the large diameter end 434 of rod 430.

Rod 430 also includes an end stop 439 to limit axial elongation of elastic element 450. End stop 439 is located on small diameter end 436 of rod 430, and has a cross-sectional area that is greater than the minimum cross-sectional area of passage 444 in housing 440. In this arrangement, end stop 439 forms an obstruction that abuts inner wall 446 of housing 440 at the narrowest section of passage 444 when the housing is moved away from the large diameter end 434 of rod 430. The end stop 439 prevents housing 440 from being moved away from the large diameter end 434 beyond a certain position, thereby limiting the amount of axial elongation of the elastic member. This can be used to prevent the spine from undergoing extreme flexion.

End stop 439 may be designed to allow a limited range of motion along the rod axis. For example, stop 439 may be positioned with respect to rod 430 and housing 440 to permit the stop to move approximately 4 mm in either direction with respect to the housing. Smaller or larger ranges of motion may also be used and are contemplated within the scope of the invention.

A number of configurations may be used for the end stop. For example, end stop 439 is shown as a cap that is threaded onto the small diameter end of rod 430. The end stop 439 can also be welded to ensure that it remains secure. Alternatively, end stop 439 can be integrally formed as a widened portion on the small diameter end of the rod 430.

Housing 440 also features a pivot point or rim 441 that is located closer to the end of the housing facing the large end of the rod 430. Because this pivot rim is closer to the large end of the rod 430, the length of the small end of the rod need not be as long. This has the benefit of shortening the overall length of the rod 430, and prevents the end of the small diameter portion of the rod from projecting out of the end of the housing during extreme compression of the elastic member.

It is noted that the middle section 438 of the rod 430, which provides the stop for spring compression, is larger in diameter than the corresponding middle section of rod 30 in FIGS. 13 and 14. Therefore, middle section 438 of rod 430 will be relatively stiff compared to the middle section of rod 30, providing a comparatively higher amount of resistance to bending. If the desired biomechanics require more flexibility, alternative stop designs may be used to limit spring compression. For example, the middle section of the rod may have a small diameter as in FIGS. 13 and 14, and a cylindrical tube can be placed inside the spring to provide the stop. The tube would have an outer diameter and length the same as the midsection 438 in FIG. 22, but would be separate from the rod 430. Because the tube is separate from rod 430, the tube would not affect the flexibility of the rod.

The small diameter end of rods in accordance with the invention may be circular in cross section, as shown with rod 30. Alternatively, the small diameter end of the rod may be non-circular in shape to provide different dynamic properties. For example, the small diameter end of the rod and the passage in the housing may both have oval or elliptical cross-sectional shapes to provide resistance to higher torsional loads, as compared to circular rods and passages, which permit a greater amount of deflection in response to torsion.

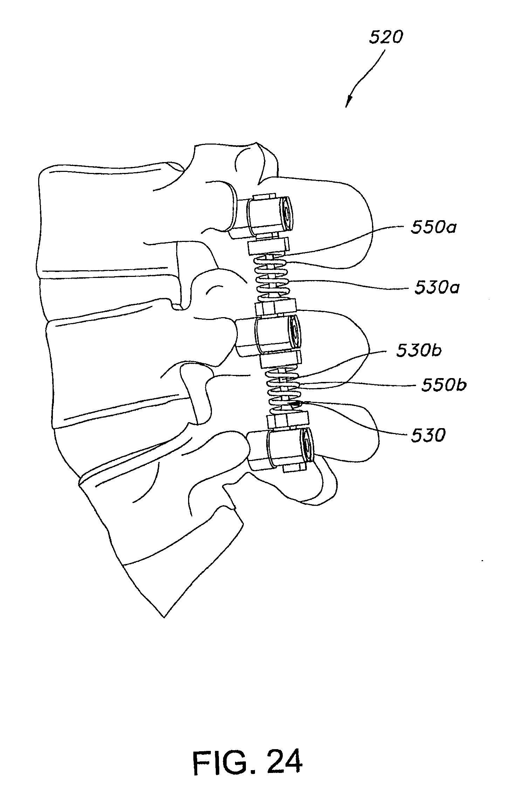

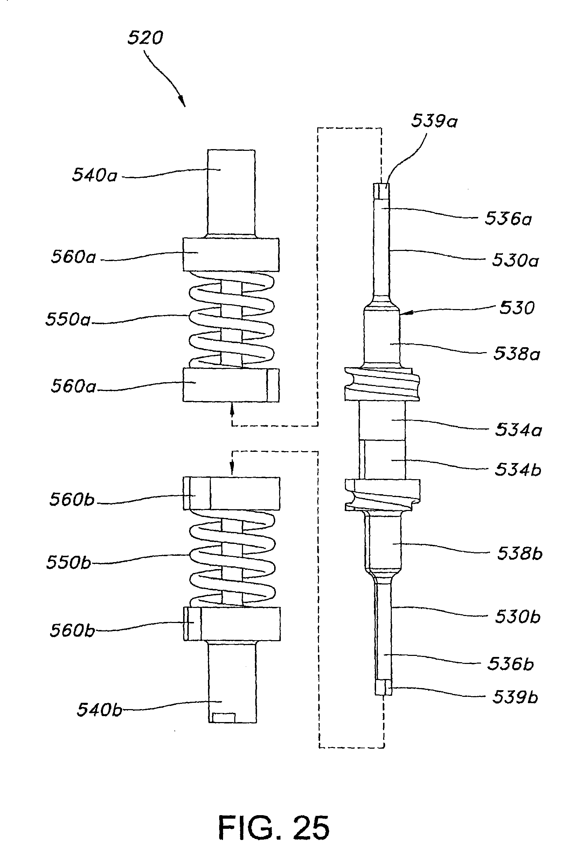

Referring now to FIGS. 24 and 25, another exemplary rod assembly 520 is shown in accordance with the invention. Rod assembly 520 is a two-level construct with many of the same component features and functionalities provided in rod assemblies 20 and 420. Whereas rod assembly 20 can be used for topping off a fused disc space (for example, where one disc space is fused and an adjacent space is allowed a limited range of motion), rod assembly 420 can be used where both instrumented levels are to remain dynamically stabilized, with controlled ranges of motion. The two levels may be stabilized with the same range of motion, or have different ranges of motion.

Rod assembly 520 includes a rod 530 with a first rod section 530a and a second rod section 530b, as shown best in FIG. 25. Rod sections 530a and 530b are identical and arranged in a mirror arrangement. Rod sections 530a, 530b have large diameter portions 534a, 534b, small diameter ends 536a, 536b, and middle sections 538a, 538b. The diameters of middle sections 538a, 538b are less than the diameters of large diameter portions 534a, 534b but greater than the diameters of small diameter ends 536a, 536b. Rod section 530a is adapted for assembly with a housing 540a and elastic element 550a using collars 560a. Similarly, rod section 530b is adapted for assembly with a housing 540b and elastic element 550b using collars 560b. During operation, the middle sections 538a, 538b each provide an end stop to limit compression of elastic members 550a and 550b, similar to the manner described in connection with middle section 438 shown in FIG. 23. Rod sections 530a and 530b also include end stops 539a, 539b at their free ends to limit axial elongation of the elastic members 550a and 550b, respectively, similar to end stop 439 in FIG. 23.

Although the invention is illustrated and described herein with reference to specific embodiments, the invention is not intended to be limited to the details shown. Rather, various modifications may be made in the details within the scope and range of equivalents of the claims and without departing from the invention.

## Claims

1. An assembly for dynamic stabilization of the spine, the assembly comprising:
a rod comprising a first end having a first diameter and a second end having a second diameter smaller than the first diameter;
a hollow housing forming a passage that receives the second end of the rod; and
an elastic member connecting the first end of the rod with the housing.

2. The assembly of claim 1, wherein the elastic member circumscribes at least a portion of the rod between the first end and the second end.

3. The assembly of claim 1 or 2, wherein the elastic member comprises a coiled spring.

4. The assembly of claim 3, wherein at least one of the rod and the housing comprises a spiral-shaped groove adapted to receive an end portion of the coiled spring.

5. The assembly according to anyone of the preceding claims comprising at least one locking collar connecting the elastic member with one of the rod and the housing.

6. The assembly according to anyone of the preceding claims, wherein the elastic member comprises a first end and a second end opposite the first end, the assembly further comprising a first locking collar coupling the first end of the elastic member with the rod and a second locking collar coupling the second end of the elastic member with the housing.

7. The assembly according to anyone of the preceding claims, wherein the passage comprises an inner wall that is offset from the axis of the rod by an angle between about 0 degrees and about 15 degrees.

8. The assembly of claim 7, wherein the angle is about 7 degrees.

9. The assembly of claim 7, wherein the angle is about 10 degrees.

10. The assembly according to anyone of the preceding claims comprising a flexible sheath extending over the elastic member between the first end of the rod and the housing.

11. The assembly according to anyone of the preceding claims, further comprising:
a first pedicle screw having a rod receiving slot, the first end of the rod being supported in the rod receiving slot of the first pedicle screw; and
a second pedicle screw having a rod receiving slot, the housing being supported in the rod receiving slot of the second pedicle screw.

12. The assembly according to anyone of claims 5 to 10, wherein the at least one locking collar comprises a bore that receives a portion of the elastic member.

13. The assembly of claim 12, wherein the elastic member comprises a coiled spring and the bore of the locking collar comprises a groove receiving a portion of the coiled spring.

14. An assembly for dynamic stabilization of the spine, the assembly comprising:
a rod having a longitudinal axis, the rod comprising:
a first end having a first diameter; and
a second end having a second diameter smaller than the first diameter; and
a sleeve coupled to the rod that circumscribes at least a portion of the second end of the rod, the sleeve comprising:
a cylindrical housing; and
an elastic section connected between the cylindrical housing and the first end of the rod, the elastic section being expandable parallel to the longitudinal axis of the rod.
